# FASCICULE DE BREVET EUROPEEN

(11) **EP 2 909 185 B1**
(45) Date de publication et mention de la délivrance du brevet: **07.12.2016**
(21) Numéro de dépôt: 13789199.0
(22) Date de dépôt: 14.10.2013
(51) Int. Cl.: G01N 33/58, A61K 47/48, C07D 263/42

(54) **RÉACTIFS DE COUPLAGE MULTIFONCTIONNELS À FONCTION AZLACTONE**
MULTIFUNKTIONELLE KOPPLUNGSREAGENZIEN MIT EINER AZLACTONFUNKTION
MULTIFUNCTIONAL COUPLING REAGENTS HAVING AN AZLACTONE FUNCTION

(30) Priorité: 18.10.2012 FR 1259941
(43) Date de publication de la demande: 26.08.2015
(73) Titulaire: CENTRE NATIONAL DE LA RECHERCHE SCIENTIFIQUE, 75016 Paris (FR); Université du Mans, 72000 Le Mans (FR)
(72) Inventeur: FONTAINE, Laurent, 72000 Le Mans (FR); HO, The Hien, 14200 Herouville Saint Clair (FR); PASCUAL, Sagrario, F-72000 Le Mans (FR); MONTEMBAULT, Véronique, F-72000 Le Mans (FR)
(74) Mandataire: Pontet Allano & Associes
(86) Numéro de dépôt international: PCT/EP2013/071430
(87) Numéro de publication internationale: WO 2014/060357

(56) Documents cités:
- WO-A1-93/25594
- WO-A2-2004/081538
- JORDI SOLÀ ET AL: "Nanometer-Range Communication of Stereochemical Information by Reversible Switching of Molecular Helicity", ANGEWANDTE CHEMIE INTERNATIONAL EDITION, vol. 49, no. 38, 4 août 2010 (2010-08-04), pages 6836-6839, XP055063212, ISSN: 1433-7851, DOI: 10.1002/anie.201001130 cité dans la demande

## Description

La présente invention a pour objet de nouveaux composés multifonctionnels, et leur utilisation en chimie click dans un procédé de couplage entre une biomolécule et une molécule cible.

Le domaine de l'invention est la conception et la synthèse de molécules possédant au moins deux fonctions chimiques réactives distinctes permettant de réaliser des réactions de chimie dite « click » entre des biomolécules renfermant des groupements amine primaire telles que des protéines, des acides nucléiques ou certains polysaccharides.

Il existe à l'heure actuelle une très forte demande dans le domaine de la bioconjugaison ou ligation de biomolécules avec des polymères naturels ou synthétiques, en raison des applications potentielles de tels bioconjugués en biologie, biochimie, biotechnologies et nanomédecine.

L'immense complexité et diversité de la vie représente un énorme défi pour les scientifiques tentant de découvrir sa base chimique. Le décryptage de la composition génétique de différents organismes n'est pas complètement utile si elle n'est pas accompagnée par la connaissance de la fonction des protéines encodées. La bioconjugaison qui consiste à coupler deux biomolécules par une liaison covalente est un moyen d'atteindre ce but.

En particulier, la bioconjugaison représente une approche intéressante pour comprendre la régulation et la fonction biologique de certaines protéines et certains biopolymères par la fixation de ligands. Cette approche consiste à accrocher à des molécules d'intérêt biologique de petites molécules synthétiques ou naturelles qui peuvent fonctionner comme des sondes pour suivre la fixation du ligand. De telles sondes incluent par exemple des molécules fluorescentes, la biotine et des sondes de Résonance Magnétique Nucléaire (RMN). Cette technique offre la possibilité de tester rapidement un grand nombre de ligands potentiels. Une autre approche consiste à introduire des groupements fonctionnels synthétiques sur des biomolécules, étape suivie par leur immobilisation sur des surfaces par une réaction chimiosélective. La biomolécule immobilisée peut être exposée ensuite à différentes molécules pour identifier ses ligands. Les puces à ADN et les puces à protéines sont des exemples courants d'une telle approche.

La bioconjugaison permet également des essais biochimiques, des applications de diagnostic par la détection qualitative et quantitative d'analytes dans des échantillons cliniques, des applications dans le domaine de l'imagerie *in vivo,* par exemple par la fixation d'agents de contraste conjugués à des anticorps, et l'utilisation d'enzymes immobilisées utilisées comme catalyseurs industriels. D'autres molécules moins courantes utilisées en bioconjugaison sont les oligosaccharides, les polymères synthétiques comme le polyéthylèneglycol (PEG), et les nanotubes de carbone.

La difficulté à contourner dans les réactions de bioconjugaison est la perte de la fonction biologique de la molécule cible à cause d'un mauvais contrôle du site où a lieu la modification. Les méthodes de bioconjugaison développées récemment sont plus spécifiques à un site et entrainent des perturbations minimales au niveau de la forme active de la biomolécule. De plus, certaines biomolécules immobilisées peuvent présenter une capacité de fixation de ligand accrue.

Les procédés de bioconjugaison chimique les plus courants reposent sur des résidus cystéine ou lysine. Des méthodes plus récentes emploient aussi des groupes fonctionnels synthétiques, comme les oléfines.

La dérivation de protéines par un groupe thiolate d'un résidu cystéine est une méthode usuelle de bioconjugaison, car les thiolates sont des nucléophiles potentiels en solution aqueuse. Des groupes fonctionnels thiol-réactifs incluent les iodoacétamides, les maléimides et les disulfures. A titre d'exemple, les iodoacétamides sont utilisés assez classiquement dans des tests de détermination de la présence de cystéines libres dans les protéines. Plus récemment, des groupes iodoacétamides ont été utilisés pour marquer des protéines avec des fluorophores, ou pour immobiliser des protéines.

Les liaisons amides ayant une grande stabilité, ce sont des cibles de choix pour la bioconjugaison. Par exemple, une protéine peut être traitée avec une petite molécule ou surface présentant une liaison ester activée pour former une liaison amide avec les groupes amines des lysines et des extrémités N-terminales. La ligation chimique native et la ligation Staudinger sont deux approches récentes pour générer des liaisons amides à des sites spécifiques d'une protéine donnée.

Une autre cible pour la bioconjugaison est la synthèse aisée de doubles liaisons carbone-azote par condensation de bases azotées avec des aldéhydes ou des cétones dans des solutions aqueuses à pH neutre. On peut ainsi synthétiser des oximes (C=N-O) et des hydrazones (C=N-N) qui sont plus stables que de simples imines (C=N). Ainsi, les glucides sont susceptibles d'être modifiés par des doubles liaisons carbone-azote puisque leurs groupes hydroxyles peuvent être oxydés facilement en aldéhydes. Alternativement, des cétones peuvent être introduites sur des sucres présents à la surface des cellules par biosynthèse. Des sucres immobilisés par des liaisons oxime ont été utilisés pour produire des puces à sucres. De nombreux exemples d'oligonucléotides conjugués par des liaisons oxime ou hydrazone sont donnés dans l'art antérieur. Comme application, on peut citer l'utilisation de puces à peptides par l'immobilisation de peptides permettant la détection d'anticorps dans des échantillons sanguins.

La réaction de cycloaddition de Huisgen en présence d'un catalyseur au cuivre Cu(I) est l'une des plus utilisées en bioconjugaison. Elle consiste en la réaction entre un alcyne terminal et un azoture générant un triazole disubstitué 1,4. Cette réaction a été utilisée pour de très nombreuses applications, notamment le marquage de protéines avec de petites molécules, l'immobilisation de protéines et de peptides, des applications en protéomique, l'immobilisation de sucres, la fonctionnalisation d'ADN, et la fixation de molécules fluorescentes sur des virus et des polymères bioactifs.

Ces dernières années, l'utilisation de polymères fonctionnalisés avec une fonction azlactone (ou oxazolone) pour la mise au point de matériaux fonctionnels s'est accrue. Du fait que les azlactones peuvent réagir par des réactions d'ouverture de cycle avec une grande diversité d'espèces nucléophiles, comme les amines primaires, les groupes hydroxyles, et les fonctions thiols, les matériaux fonctionnalisés avec une fonction azlactone peuvent servir de plateformes réactives pour l'introduction post-synthèse ou post-fabrication d'une large gamme de fonctions chimiques à des polymères solubles, des supports insolubles et des surfaces. La réactivité de ce cycle électrophile est telle qu'une réaction d'ouverture conduit à la formation d'un lieur (linker) fonctionnel peu réactif. En effet, il est constitué soit de deux liaisons amides si le nucléophile est une amine primaire, soit d'une liaison amide et d'une liaison ester si le nucléophile est un alcool, soit d'une liaison amide et d'une liaison thioester si le nucléophile est un thiol. Elles encadrent un centre tétrasubstitué très encombré. Le site global constituant le lieur (linker) est donc peu réactif.

La dernière décennie a vu une augmentation importante à la fois du nombre et de la variété d'applications exploitant les propriétés et la réactivité de tels polymères fonctionnalisés. Diverses études ont montré l'intérêt des supports à fonction azlactone tels que des supports solubles, des films, des monolithes et des supports insolubles sous forme de billes. Les applications de ces supports, en relation avec la composition et la morphologie du support, restent toutefois limitées et se résument principalement à l'immobilisation d'enzymes, de catalyseurs, de ligands ou au piégeage d'amines.
Par exemple, concernant les monolithes, deux applications possibles, en lien avec leur structure, ont été mises en avant : les monolithes hydrophiles sont utilisés en milieu aqueux pour immobiliser des enzymes comme la trypsine ou l'albumine bovine sérique (BSA) et les monolithes hydrophobes sont utilisés pour le piégeage d'amines en milieu organique. De la même manière que pour les monolithes, les supports réticulés insolubles à fonctionnalité azlactone sous forme de billes présente deux applications majeures : l'immobilisation d'enzymes et le piégeage d'amines. Ainsi SOLA J. et al. (Angew. Chem. Int. Ed., 2010, 49, 6836-6839) divulguent des azotures qui sont utilisés pour étudier les modifications des structures en hélice d'oligomères peptidomimétiques à base d'acide aminoisobutyrique (Aib). La demande internationale WO 2004/081538 décrit des azlactones qui sont utilisées comme intermédiaires pour la synthèse d'homopolymères polyoxazolone aptes à être conjuguées avec différents agents actifs et la demande internationale WO 93/25594 décrit des groupes azlactones fixés sur des supports et utilisés pour fixer des agents actifs.

Dans la recherche thérapeutique actuelle, de nouvelles méthodologies ont été développées pour accéder plus rapidement à une grande diversité de composés. En particulier, la ligation chimique par chimie « click » a été proposée par Sharpless en 2001 (H.C. Kolb, M.G. Finn et K.B. Sharpless (2001). "Click Chemistry: Diverse Chemical Function from a Few Good Reactions". Angewandte Chemie International Edition 40 (11): 2004-2021) pour générer des structures originales différentes des pharmacophores classiques. Une réaction pourra être considérée comme « click » si elle répond aux critères suivants :
- modularité
- stéréosélectivité
- insensibilité à l'oxygène et à l'eau
- pureté et rendement élevés.

La chimie « click » permet donc de développer un ensemble de réactions univoques et modulables, pouvant être mises en oeuvre dans des conditions douces, sans purification fastidieuse, sans formation de sous-produit, possédant un large spectre de substrats, physiologiquement stables et/ou compatibles avec les milieux biologiques et conduisant avec économie d'atomes à de hauts rendements. Les principales réactions « click » consistent à former des liaisons carbone-hétéroatome énergétiquement très favorables, notamment une réaction nucléophile d'ouverture de cycle ou une réaction de cycloaddition. Un type de réaction largement représentée en chimie « click » est la cycloaddition alcyne-azoture catalysée au Cu(I) mentionnée ci-dessus. Lorsque les réactions « click » sont compatibles avec les fonctions chimiques et les milieux biologiques, que ce soit *in vitro* ou *in vivo,* ces réactions sont dites bio-orthogonales.

Bien qu'il existe de nombreux réactifs aptes à être utilisés dans ces réactions de chimie click, il existe un besoin de nouveaux réactifs permettant des réactions efficaces et rapides en milieu aqueux, sans formation de sous-produits difficiles à éliminer et/ou toxiques.

Un but de l'invention est donc de proposer une famille de nouveaux agents réactifs de couplage multifonctionnels utilisables pour l'association par liaisons covalentes de biomolécules telles que des protéines, des peptides, de l'ADN, certains polysaccharides, avec une ou plusieurs autre(s) biomolécule(s), un polymère naturel ou synthétique, ou une surface réactive. Ces nouveaux composés présentent un intérêt tout particulier pour la chimie « click » appliquée à la biologie et la synthèse chimique.

Un autre but de l'invention est également de pallier les inconvénients de l'état de la technique en proposant de nouveaux réactifs de couplage :
- permettant des associations par des processus bio-orthogonaux, à la fois *in vitro* et *in vivo,*
- dont les fonctionnalités réactives sont également orthogonales entre elles,
- dont la réactivité élevée est compatible avec les milieux biologiques,
- présentant une résistance à l'hydrolyse en milieu aqueux,
- sans formation de sous-produit toxique ou difficile à éliminer,
- applicables à une large gamme de macromolécules biologiques ou synthétiques,
- conduisant à des liaisons chimiques robustes et compatibles avec une vaste gamme de fonctionnalités chimiques ainsi qu'avec les milieux biologiques et les organismes vivants, *in vitro* et *in vivo.*

Certains composés répondant à la formule générale (I) 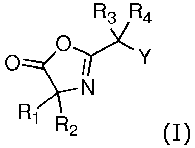 dans laquelle
- R₁ et R₂ représentent indépendamment l'un de l'autre un groupe (C₁-C₁₀)alkyle, un groupe (C₂-C₆)cycloalkyle, un groupe aryle, un groupe aryl(C₁-C₁₀)alkyle, ou un groupe hétérocyclique,
- R₃ et R₄ représentent indépendamment l'un de l'autre un atome d'hydrogène, un groupe (C₁-C₁₀)alkyle, un groupe aryle ou un groupe aryl(C₁-C₁₀)alkyle, et
- Y représente une fonction réactive activable par chimie click, choisie dans le groupe comprenant les azotures, les alcynes, les cycloalcynes et les diènes conjugués,
sont connus en tant que tels mais ne sont pas décrits comme des agents réactifs de couplage multifonctionnels (voir SOLA J. cité précédemment).

Selon la présente invention, le terme « (C₁-C₁₀)alkyle » représente un groupe hydrocarboné saturé, linéaire ou ramifié, ayant de 1 à 10 atomes de carbone, avantageusement de 1 à 5 atomes de carbone. A titre d'exemple, on peut citer les groupes méthyle, éthyle, propyle, isopropyle, butyle, isobutyle, sec-butyle, tertiobutyle, pentyle, néopentyle, isopentyle, tert-pentyle, hexyle, isohexyle, heptyle, octyle, nonyle et décyle.

Le terme « aryle » représente un groupe hydrocarboné aromatique mono- ou bicyclique comprenant de 6 à 10 atomes de carbone. A titre d'exemple on peut citer les groupes phényle et naphtyle.

Par le terme « aryl(C₁-C₁₀)alkyle », on entend un groupe alkyle ayant de 1 à 10 atomes de carbone, avantageusement de 1 à 5 atomes de carbone tel que défini précédemment et contenant un groupe aryle tel que défini ci-dessus.

Le terme « groupe hétérocyclique » représente tout hétérocycle saturé ou insaturé, comprenant de 3 à 7 atomes de carbones et contenant un à 3 hétéroatomes choisi dans le groupe constitué par l'oxygène, l'azote ou le soufre. On peut citer par exemple les groupes pipéridinyle, pyrrolidinyle, pipérazinyle, pyridyle, piridinyle, imidazolyle, furyle, morpholinyle, oxétanyle, tétrahydrofuranyle, tétrahydropyranyle, tétrahydrothiényle, et thiazolyle.

Le terme « azotures » représente les sels de l'acide azothydrique HN₃, ainsi que les azotures organiques dans lesquels un des atomes d'azote est lié de façon covalente avec un atome de carbone d'un composé organique. On peut citer comme exemples l'azoture de méthyle et l'azoture de phényle.

Le terme « alcynes » représente les hydrocarbures possédant une insaturation caractérisée par la présence d'une triple liaison carbone-carbone. On peut citer comme exemples l'éthyne, le propyne, le but-1-yne, et le but-2-yne.

On entend par « cycloalcynes » tout cycle d'atomes de carbone contenant une ou plusieurs triples liaisons, comme le cyclooctyne.

Le terme « diènes conjugués » représente des hydrocarbures possédant deux doubles liaisons séparées par une seule liaison simple. A titre d'exemple, on peut citer le butadiène, l'isoprène, le 2,3-diméthyl-1,3-butadiène, le 1,3-pentadiène, le 2-phényl-1,3-butadiène et le furane.

La première fonction chimique commune aux composés répondant à la formule (I) utilisés selon l'invention est de type azlactone (2-oxazolin-5-one). Ce groupement est connu pour réagir par ouverture de cycle avec les fonctions amine primaire sans nécessiter de catalyseur et avec formation d'un lien amide robuste dans des conditions douces, notamment en solution aqueuse, donc compatibles avec les milieux biologiques. L'intérêt majeur de cette fonction chimique comparée aux systèmes déjà décrits réside, outre sa réactivité intrinsèque conduisant à une réaction rapide et efficace en milieu aqueux grâce à sa résistance à l'hydrolyse, dans le fait que la réaction avec une amine n'entraîne pas la formation de sous-produit de réaction éventuellement toxique et difficile à éliminer du milieu réactionnel. Les fonctions amines primaires sont très répandues dans la plupart des molécules biologiques (protéines, acides nucléiques notamment) et des molécules d'intérêt biologique, ce qui assure un champ d'application très large aux réactifs de cette invention. En termes de coût, l'absence de sous-produit généré par réaction du groupe azlactone conduit de plus à une économie d'atomes. De plus, la possibilité de travailler en milieu aqueux est un atout en matière d'environnement.

L'autre fonction commune présente dans les composés répondant à la formule (I) utilisés selon l'invention correspond à un groupement apte à participer à une réaction de cycloaddition, en particulier les groupes azoture, alcyne, cycloalcyne et diène conjugué. Les réactions de cycloaddition constituent les exemples les plus répandus de chimie click, notamment la réaction de Huisgen citée précédemment, la cycloaddition [4+2] (réaction de Diels-Alder), et l'hétérocycloaddition. Ces réactions sont bio-orthogonales, c'est-à-dire qu'elles sont compatibles avec les espèces présentes dans les milieux biologiques. Elles mettent en effet en oeuvre des groupements fonctionnels qui ne sont en général ni présents, ni susceptibles de réagir avec les groupes existant au sein des biomolécules.

Les deux fonctions chimiques présentes dans les composés selon l'invention sont de plus orthogonales entre elles, c'est-à-dire qu'elles réagissent dans des conditions différentes, indépendamment les unes des autres. Il est ainsi possible de déclencher la réaction de l'un de ces deux groupements sans que l'autre ne soit transformé, permettant ainsi des réactions successives chimiosélectives.

La présente invention a pour objet des composés de formule (I) 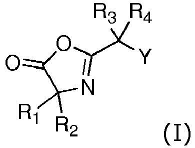 dans laquelle
- R₁ et R₂ représentent indépendamment l'un de l'autre un groupe (C₁-C₁₀)alkyle, un groupe (C₂-C₆)cycloalkyle, un groupe aryle, un groupe aryl(C₁-C₁₀)alkyle, ou un groupe hétérocyclique,
- R₃ et R₄ représentent indépendamment l'un de l'autre un atome d'hydrogène, un groupe (C₁-C₁₀)alkyle, un groupe aryle ou un groupe aryl(C₁-C₁₀)alkyle, et
Y représente :
(i) un groupe N₃, ou
(ii) un groupe (C₁-C₁₀)alkyl-N₃, ou
(iii) un groupe aryl-N₃, ou
(iv) un groupe aryl(C₁-C₁₀)alkyl-N₃, ou
(v) un groupe (C₁-C₁₀)alkyl-C≡C-R₅, ou
(vi) un groupe aryl-OC-R₅, ou
(vii) un groupe aryl(C₁-C₁₀)alkyl- C≡C-R₅, ou
(viii) un groupe -O-C(O)-(CH₂)ₙ-C≡CR₅, avec n entier compris entre 1 et 10, notamment un groupe -O-C(O)-(CH₂)ₙ-C≡CH, avec n compris entre 1 et 5, avec R₅ représentant
   (a) soit un atome d'hydrogène,
   (b) soit un groupe (C₁-C₁₀)alkyle,
   (c) soit un groupe aryle,
   (d) soit un groupe aryl(C₁-C₁₀)alkyle,
      tous ces groupes pouvant renfermer deux doubles liaisons conjuguées, éventuellement dans un cycle,
   (e) soit un groupe protecteur de la triple liaison.

Dans les définitions (i) à (viii), les termes (C₁-C₁₀)alkyle, aryle et aryl(C₁-C₁₀)alkyle renvoient aux définitions données précédemment.

Comme exemples de groupe (C₁-C₁₀)alkyle, aryle ou aryl(C₁-C₁₀)alkyle, pouvant renfermer deux doubles liaisons conjuguées, éventuellement dans un cycle, on peut citer notamment le 1,3-butadiènyle, le cyclopentadiènyle, le pyrrolyle et le furyle.

Tous les groupements cités possèdent en commun l'aptitude à donner une réaction de cycloaddition dipolaire [3+2] ou de cycloaddition ou hétérocycloaddition [4+2].

Les groupes protecteurs de la triple liaison sont bien connus de l'homme du métier. Comme exemples, on peut citer les trialkysilyles, notamment le triméthylsilyle, le triéthylsilyle, le tert-butyldiméthylsilyle et le benzyldiméthylsilyle.

Selon un autre mode de réalisation avantageux de l'invention, les composés de formule (I) sont ceux pour lesquels R₁ et R₂ représentent chacun un groupe (C₁-C₁₀)alkyle, R₃ représente un atome d'hydrogène, R₄ représente un groupe méthyle, et Y représente soit un groupe N₃, soit un groupe -O-C(O)-(CH₂)₃-C≡CH.

Avantageusement, les groupes R₁, R₂ et R₄ représentent chacun un groupe méthyle, R₃ un atome d'hydrogène et Y un groupe N₃.

Selon un autre mode de réalisation avantageux de l'invention, R₁, R₂ et R₄ désignent chacun un groupe méthyle, R₃ un atome d'hydrogène et Y un groupe -O-C(O)-(CH₂)₃-C≡CH.

Un autre objet de l'invention est un procédé de couplage entre une biomolécule et une molécule cible choisie dans le groupe comprenant une molécule d'intérêt biologique, un polymère naturel ou synthétique, et une surface réactive, ledit procédé mettant en oeuvre un composé de formule (I) dans laquelle
- R₁ et R₂ représentent indépendamment l'un de l'autre un groupe (C₁-C₁₀)alkyle, un groupe (C₂-C₆)cycloalkyle, un groupe aryle, un groupe aryl(C₁-C₁₀)alkyle, ou un groupe hétérocyclique,
- R₃ et R₄ représentent indépendamment l'un de l'autre un atome d'hydrogène, un groupe (C₁-C₁₀)alkyle, un groupe aryle ou un groupe aryl(C₁-C₁₀)alkyle, et
- Y représente une fonction réactive activable par chimie click, choisie dans le groupe comprenant les azotures, les alcynes, les cycloalcynes et les diènes conjugués,
   et comprenant les étapes suivantes :
   - mise en contact dudit composé de formule (I) avec une biomolécule,
   - mise en contact dudit composé de formule (I) lié avec la biomolécule avec la molécule cible et si nécessaire,
   - isolement du produit de couplage.

Dans un mode de réalisation avantageux de l'invention, le procédé de couplage met en oeuvre un composé formule (I) dans laquelle
- R₁ et R₂ représentent indépendamment l'un de l'autre un groupe (C₁-C₁₀)alkyle, un groupe (C₂-C₆)cycloalkyle, un groupe aryle, un groupe aryl(C₁-C₁₀)alkyle, ou un groupe hétérocyclique,
- R₃ et R₄ représentent indépendamment l'un de l'autre un atome d'hydrogène, un groupe (C₁-C₁₀)alkyle, un groupe aryle ou un groupe aryl(C₁-C₁₀)alkyle, et
Y représente :
(i) un groupe N₃, ou
(ii) un groupe (C₁-C₁₀)alkyl-N₃, ou
(iii) un groupe aryl-N₃, ou
(iv) un groupe aryl(C₁-C₁₀)alkyl-N₃, ou
(v) un groupe (C₁-C₁₀)alkyl-C≡C-R₅, ou
(vi) un groupe aryl-C≡C-R₅, ou
(vii) un groupe aryl(C₁-C₁₀)alkyl- C≡C-R₅, ou
(viii) un groupe -O-C(O)-(CH₂)ₙ-C≡CR₅, avec n entier compris entre 1 et 10, notamment un groupe -O-C(O)-(CH₂)ₙ-C≡CH, avec n compris entre 1 et 5, avec R₅ représentant
   (a) soit un atome d'hydrogène,
   (b) soit un groupe (C₁-C₁₀)alkyle,
   (c) soit un groupe aryle,
   (d) soit un groupe aryl(C₁-C₁₀)alkyle,
      tous ces groupes pouvant renfermer deux doubles liaisons conjuguées, éventuellement dans un cycle,
   (e) soit un groupe protecteur de la triple liaison.

Par « surface réactive », on entend un matériau organique ou inorganique possédant à sa surface des fonctions chimiques aptes à réagir avec les molécules de la présente invention. On peut citer par exemple, une surface (film, tissu, ...) de polypropylène ou polyéthylène possédant des fonctions amine en surface.

Selon un mode de réalisation avantageux du procédé selon l'invention, la/les biomolécules mise(s) en jeu dans ledit procédé de couplage sont choisies dans le groupe comprenant des protéines, des peptides, de l'ADN, des marqueurs biologiques, des hormones, des vitamines, des anticorps, des polyamines, des monosaccharides, des oligosaccharides et des polysaccharides, et des molécules d'intérêt biologique du type médicament comme des anticancéreux, des anti-viraux, ou marqueur comme les marqueurs fluorescents ou radioactifs.

Un autre objet de l'invention est un réactif de diagnostic mettant en oeuvre au moins un composé de formule (I), notamment un composé pour lequel
- R₁ et R₂ représentent indépendamment l'un de l'autre un groupe (C₁-C₁₀)alkyle, un groupe (C₂-C₆)cycloalkyle, un groupe aryle, un groupe aryl(C₁-C₁₀)alkyle, ou un groupe hétérocyclique,
- R₃ et R₄ représentent indépendamment l'un de l'autre un atome d'hydrogène, un groupe (C₁-C₁₀)alkyle, un groupe aryle ou un groupe aryl(C₁-C₁₀)alkyle, et
Y représente :
(i) un groupe N₃, ou
(ii) un groupe (C₁-C₁₀)alkyl-N₃, ou
(iii) un groupe aryl-N₃, ou
(iv) un groupe aryl(C₁-C₁₀)alkyl-N₃, ou
(v) un groupe (C₁-C₁₀)alkyl-C≡C-R₅, ou
(vi) un groupe aryl-C≡C-R₅, ou
(vii) un groupe aryl(C₁-C₁₀)alkyl- C≡C-R₅, ou
(viii) un groupe -O-C(O)-(CH₂)ₙ-C≡CR₅, avec n entier compris entre 1 et 10, notamment un groupe -O-C(O)-(CH₂)ₙ-C≡CH, avec n compris entre 1 et 5, avec R₅ représentant
   (a) soit un atome d'hydrogène,
   (b) soit un groupe (C₁-C₁₀)alkyle,
   (c) soit un groupe aryle,
   (d) soit un groupe aryl(C₁-C₁₀)alkyle,
      tous ces groupes pouvant renfermer deux doubles liaisons conjuguées, éventuellement dans un cycle,
   (e) soit un groupe protecteur de la triple liaison.

L'invention a également pour objet l'utilisation des composés de formule (I) dans laquelle
- R₁ et R₂ représentent indépendamment l'un de l'autre un groupe (C₁-C₁₀)alkyle, un groupe (C₂-C₆)cycloalkyle, un groupe aryle, un groupe aryl(C₁-C₁₀)alkyle, ou un groupe hétérocyclique,
- R₃ et R₄ représentent indépendamment l'un de l'autre un atome d'hydrogène, un groupe (C₁-C₁₀)alkyle, un groupe aryle ou un groupe aryl(C₁-C₁₀)alkyle, et
- Y représente une fonction réactive activable par chimie click, choisie dans le groupe comprenant les azotures, les alcynes, les cycloalcynes et les diènes conjugués,
comme réactif de diagnsotic et une méthode de diagnostic mettant en oeuvre l'ensemble des composés de formule (I).

En effet, les agents de couplage présentent une utilité dans le domaine du diagnostic, notamment dans la détection de réactions du type ligand-anti-ligand tel que antigène-anticorps ou protéine-protéine, car ils permettent de coupler directement une molécule d'intérêt biologique, telle qu'un antigène, à une molécule dite de révélation, telle qu'une enzyme. La liaison de la molécule biologique d'intérêt à une autre molécule, telle qu'un anticorps, est mise en évidence grâce à la molécule de révélation. Il peut être également utile de coupler une sonde fluorescente à une molécule d'intérêt biologique pour sa détection par imagerie fonctionnelle.

Un autre objet de l'invention est un kit pour la mise en oeuvre d'un procédé de couplage et de bioconjugaison comprenant au moins un composé de formule (I), notamment ceux pour lesquels
- R₁ et R₂ représentent indépendamment l'un de l'autre un groupe (C₁-C₁₀)alkyle, un groupe (C₂-C₆)cycloalkyle, un groupe aryle, un groupe aryl(C₁-C₁₀)alkyle, ou un groupe hétérocyclique,
- R₃ et R₄ représentent indépendamment l'un de l'autre un atome d'hydrogène, un groupe (C₁-C₁₀)alkyle, un groupe aryle ou un groupe aryl(C₁-C₁₀)alkyle, et
Y représente :
(i) un groupe N₃, ou
(ii) un groupe (C₁-C₁₀)alkyl-N₃, ou
(iii) un groupe aryl-N₃, ou
(iv) un groupe aryl(C₁-C₁₀)alkyl-N₃, ou
(v) un groupe (C₁-C₁₀)alkyl-C≡C-R₅, ou
(vi) un groupe aryl-C≡C-R₅, ou
(vii) un groupe aryl(C₁-C₁₀)alkyl-C≡C-R₅, ou
(viii) un groupe -O-C(O)-(CH₂)ₙ-C≡CR₅, avec n entier compris entre 1 et 10, notamment un groupe -O-C(O)-(CH₂)ₙ-C≡CH, avec n compris entre 1 et 5, avec R₅ représentant
   (a) soit un atome d'hydrogène,
   (b) soit un groupe (C₁-C₁₀)alkyle,
   (c) soit un groupe aryle,
   (d) soit un groupe aryl(C₁-C₁₀)alkyle,
      tous ces groupes pouvant renfermer deux doubles liaisons conjuguées, éventuellement dans un cycle,
   (e) soit un groupe protecteur de la triple liaison.

Un autre objet de l'invention est un procédé de séparation, de détection et/ou de caractérisation d'au moins une molécule d'intérêt susceptible d'être présente dans un milieu, comprenant au moins une étape de mise en oeuvre d'un kit selon l'invention ou d'au moins un composé de formule (I) dans laquelle
- R₁ et R₂ représentent indépendamment l'un de l'autre un groupe (C₁-C₁₀)alkyle, un groupe (C₂-C₆)cycloalkyle, un groupe aryle, un groupe aryl(C₁-C₁₀)alkyle, ou un groupe hétérocyclique,
- R₃ et R₄ représentent indépendamment l'un de l'autre un atome d'hydrogène, un groupe (C₁-C₁₀)alkyle, un groupe aryle ou un groupe aryl(C₁-C₁₀)alkyle, et
- Y représente une fonction réactive activable par chimie click, choisie dans le groupe comprenant les azotures, les alcynes, les cycloalcynes et les diènes conjugués,

Un autre objet de l'invention est une composition comprenant un composé de formule (I) dans lequel
- R₁ et R₂ représentent indépendamment l'un de l'autre un groupe (C₁-C₁₀)alkyle, un groupe (C₂-C₆)cycloalkyle, un groupe aryle, un groupe aryl(C₁-C₁₀)alkyle, ou un groupe hétérocyclique,
- R₃ et R₄ représentent indépendamment l'un de l'autre un atome d'hydrogène, un groupe (C₁-C₁₀)alkyle, un groupe aryle ou un groupe aryl(C₁-C₁₀)alkyle, et
Y représente :
(i) un groupe N₃, ou
(ii) un groupe (C₁-C₁₀)alkyl-N₃, ou
(iii) un groupe aryl-N₃, ou
(iv) un groupe aryl(C₁-C₁₀)alkyl-N₃, ou
(v) un groupe (C₁-C₁₀)alkyl-C≡C-R₅, ou
(vi) un groupe aryl-C≡C-R₅, ou
(vii) un groupe aryl(C₁-C₁₀)alkyl- C≡C-R₅, ou
(viii) un groupe -O-C(O)-(CH₂)ₙ-C≡CR₅, avec n entier compris entre 1 et 10, notamment un groupe -O-C(O)-(CH₂)ₙ-C≡CH, avec n compris entre 1 et 5, avec R₅ représentant
   (a) soit un atome d'hydrogène,
   (b) soit un groupe (C₁-C₁₀)alkyle,
   (c) soit un groupe aryle,
   (d) soit un groupe aryl(C₁-C₁₀)alkyle,
      tous ces groupes pouvant renfermer deux doubles liaisons conjuguées, éventuellement dans un cycle,
   (e) soit un groupe protecteur de la triple liaison.
en association avec un milieu aqueux ou organique.

Comme milieu organique, on peut citer par exemple le diméthylsulfoxyde (DMSO) compatible avec les cultures cellulaires.

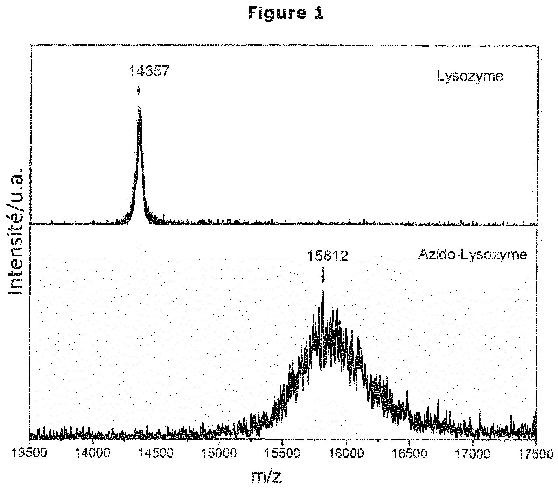

L'invention est illustrée par les exemples 1 à 6 et la figure 1 qui suivent.

Les exemples 1, 2 et 4, 5 illustrent la synthèse de composés selon l'invention et les exemples 3 et 6 le procédé de couplage entre un composé de l'invention et une biomolécule : le lysosyme.

La figure 1 représente le spectre d'analyse MALDI-TOF du conjugué lysozyme-2-(1-azidoéthyl)-4,4-diméthyloxazol-5(4H)-one obtenu selon le procédé de couplage décrit dans l'exemple 3.

### Exemple 1 : 2-(1-azidoéthyl)-4,4-diméthyloxazol-5(4H)-one

La 2-(1-azidoéthyl)-4,4-diméthyloxazol-5(4H)-one est préparée selon le schéma 1 ci-dessous.

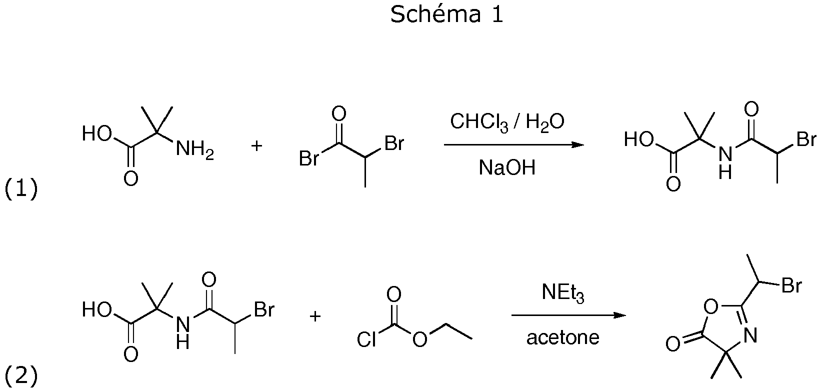

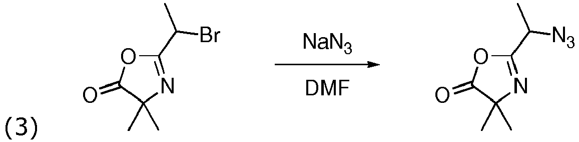

### 1.1. 2-(1-bromoéthyl)-4,4-diméthyloxazol-5(4H)-one :

Cet intermédiaire de synthèse est préparé selon la procédure décrite par K.M. Lewandowski *et al.* dans le brevet US 6 762 257 B1 (étapes 1 et 2 du schéma 1)

### 1.2. 2-(1-azidoéthyl)-4,4-diméthyloxazol-5(4H)-one.

La 2-(1-azidoéthyl)-4,4-diméthyloxazol-5(4H)-one est ensuite obtenue (étape 3) selon le protocole ci-dessous.

Dans un ballon d'une contenance de 25 mL muni d'un barreau aimanté, on prépare sous atmosphère d'argon une solution de 0,65 g (0,01 mol) d'azoture de sodium dans 4 mL de diméthylformamide (DMF) anhydre que l'on refroidit à l'aide d'un bain de glace. On verse goutte-à-goutte sur ce mélange une solution comprenant 2,20 g, (0,01 mol) de 2-(1-bromoéthyl)-4,4-diméthyloxazol-5(4H)-one, préparée à l'étape 1.1., dans 2 mL de DMF anhydre et on laisse sous agitation pendant 2 h à 0°C. On laisse revenir à température ambiante sous agitation pendant 24 h. Le solvant est évaporé sous pression réduite et le résidu est repris par l'acétate d'éthyle puis la solution est filtrée pour éliminer le bromure de sodium. Le filtrat est versé dans une ampoule à décanter et la solution est lavée avec HCl dilué (5%) puis NaHCO₃ aqueuse saturée. La phase organique est séchée sur MgSO₄, filtrée et concentrée sous vide. L'huile jaune obtenue est purifiée par chromatographie sur colonne de silice avec successivement le n-hexane puis un mélange n-hexane : acétate d'éthyle 8:2 v/v pour fournir une huile (0,57 g, 31%) qui cristallise au froid.

La 2-(1-azidoéthyl)-4,4-diméthyloxazol-5(4H)-one est caractérisée par résonance magnétique nucléaire du proton (RMN ¹H) et par résonance magnétique nucléaire du carbone 13 (RMN ¹³C).
RMN ¹H (400 MHz, CDCl₃, δ ppm) : 1,46 ppm (s, 6H, -C(CH₃)₂), 1,56 ppm (d, 3H, -CH(CH₃)N₃), 4,27 ppm (t, 1H, CH(CH₃)N₃).
RMN ¹³C (400 MHz, CDCl₃, δ ppm) : 16,32 ppm (-C(CH₃)N₃), 24,46 ppm (C(CH₃)₂), 53,76 ppm (-C(CH₃)N₃), 65,59 ppm (-C(CH₃)₂), 161,56 ppm (C=N), 180,11 ppm (C=O).

### Exemple 2 : 1-(4,4-diméthyl-5-oxo-4,5-dihydrooxazol-2-yl)éthyl hex-5-ynoate

Le 1-(4,4-diméthyl-5-oxo-4,5-dihydrooxazol-2-yl)éthyl hex-5-ynoate est préparé selon le schéma 2 ci-dessous.

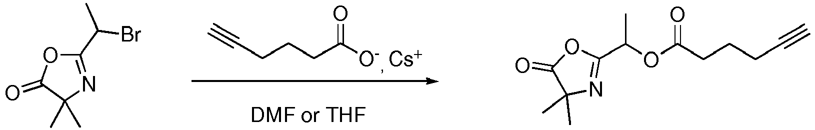

La 2-(1-bromoéthyl)-4,4-diméthyloxazol-5(4H)-one est préparée selon l'exemple 1.1.

### 2.2 1-(4,4-diméthyl-5-oxo-4,5-dihydrooxazol-2-yl)éthyl hex-5-ynoate

### 2.2.1. Sel de césium de l'acide 5-hexynoique.

Le sel de césium de l'acide 5-hexynoique est préparé par réaction du carbonate de césium (4,10 g, 0,0126 mol) sur l'acide 5-hexynoique (4,6 g, 0,0411 mol) en solution dans le DMF (6,0 mL) pendant 16 h à température ambiante. Le mélange réactionnel est filtré, le solvant est éliminé sous pression réduite et le résidu repris par l'éther diéthylique. Après filtration, le sel de césium de l'acide 5-hexynoique (4,82 g, rendement 78%) est séché sous vide à 40°C.

### 2.2.2. 1-(4,4-diméthyl-5-oxo-4,5-dihydrooxazol-2-yl)éthyl hex-5-ynoate.

A une solution de sel de césium de l'acide 5-hexynoique (0,490 g, 2 mmol) dans le DMF anhydre (3 mL) placée dans un ballon refroidi par un bain de glace est ajoutée goutte-à-goutte une solution de 2-(1-bromoéthyl)-4,4-diméthyloxazol-5(4H)-one (0,448g, 2,03 mmol) dans le DMF (2 mL). En fin d'addition, le mélange réactionnel est laissé sous agitation à 0°C pendant 2 h puis on laisse revenir à température ambiante pendant 24 h. Le solvant est éliminé sous pression réduite puis le résidu est repris par l'acétate d'éthyle, filtré et concentré sous vide. L'huile jaune obtenue est purifiée par chromatographie sur colonne de silice pour fournir 0,310 g (61%) de 1-(4,4-diméthyl-5-oxo-4,5-dihydrooxazol-2-yl)ethyl hex-5-ynoate.

Le 1-(4,4-diméthyl-5-oxo-4,5-dihydrooxazol-2-yl)ethyl hex-5-ynoate est caractérisé par résonance magnétique nucléaire du proton (RMN ¹H) et par résonance magnétique nucléaire du carbone 13 (RMN ¹³C).
RMN ¹H (400 MHz, CDCl₃, δ ppm) : 1,46 ppm (s, 6H, -C(CH₃)₂), 1,56 ppm (d, 3H, -CH(CH₃)OCO), 1,85 ppm (quinquet, 2H, HC≡C-CH₂CH₂CH₂-O-CO-), 2,00 ppm (HC≡C-), 2,29 ppm (triplet-doublet, HC≡CH₂CH₂CH₂-OCO), 2,54 ppm (HC≡C-CH₂CH₂CH₂-O-CO-), 5,57 ppm (quadruplet, 1H, -CH₂-COOCH(CH₃)-).
RMN ¹³C (400 MHz, CDCl₃, δ ppm) : 17,01 ppm (-CH₂-COOCH(CH₃)), 17,75 ppm (HC≡C-CH₂-), 23,50 ppm (HC≡C-CH₂CH₂CH₂-), 24,42 ppm (C(CH₃)₂), 32,62 ppm(-CH₂CH₂CO)-), 65,04 ppm (C(CH₃)₂), 65,45 ppm (HC≡C-CH₂-), 69,50 ppm (-COO-CH(CH₃)-), 83,24 ppm (HC≡C-CH₂-), 162,06 ppm (C=N), 172,26 ppm (-CH₂-COO-CH(CH₃)-), 180,52 ppm (C=O)_{cycle}.

### Exemple 3: Procédé de couplage de la 2-(1-azidoéthyl)-4,4-diméthyloxazol-5(4H)-one avec le lysozyme

Le couplage d'une protéine modèle, le lysozyme, avec la 2-(1-azidoéthyl)-4,4-diméthyloxazol-5(4H)-one est décrit ci-après :

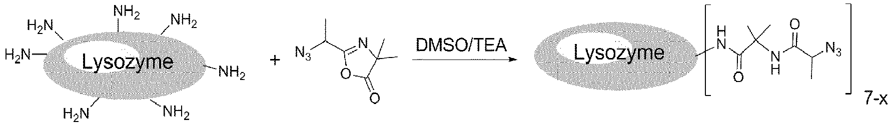

### 3.1. Mode opératoire

Dans un ballon équipé d'un barreau magnétique, une solution de lysozyme (114,2 mg, 8,0x10⁻⁶ mol) dans le diméthylsulfoxide (DMSO, 10,00 mL) est préparée à laquelle on ajoute de la triméthylamine (TEA, 0,20 mL, 1,49x10⁻³ mol). Après 15 mn d'agitation à température ambiante, une solution de 2-(1-azidoéthyl)-4,4-diméthyloxazol-5(4H)-one préparée selon l'exemple 1 (0,252 g, 1,38x10⁻³ mol) dans le DMSO (1,00 mL) est ajoutée et le mélange réactionnel est abandonné à température ambiante pendant 24h sous agitation. Après 24 h, une solution aqueuse d'HCl (HCl, 37%, 1,0 mL pour 30,0 mL de solution) est ajoutée puis la solution est dialysée avec une membrane de dialyse (seuil de coupure = MWCO = 3500) contre une solution aqueuse de méthanol (eau : méthanol = 8:2 en volumes) pendant 24 h. Le produit est ensuite récupéré par lyophilisation. Le produit final (0,012 g) est dissous dans le DMSO (0,10 mL) pour analyse par spectroscopie Infrarouge à Transformée de Fourier (IRTF). Une analyse par spectrométrie de masse à temps de vol (MALDI-TOF MS) est également réalisée.

### 3.2. Résultats

Le spectre IRTF de l'échantillon montre la présence d'une nouvelle bande d'absorption à 2110 cm⁻¹, caractéristique du groupe azoture introduit sur la protéine.

La réaction est également attestée par l'analyse en spectrométrie de masse à temps de vol (MALDI-TOF MS) qui montre qu'après réaction le pic du lysozyme de départ à m/z = 14357 a disparu au profit d'un nouveau signal centré à m/z = 15812 (figure 1).

### Exemple 4 : Synthèse du 1-(4,4-diméthyl-5-oxo-4,5-dihydro oxazol-2-yl)éthyl hexa-2,4-dienoate

Le 1-(4,4-diméthyl-5-oxo-4,5-dihydrooxazol-2-yl)éthylhexa-2,4-dienoate est préparé selon le schéma ci-dessous :

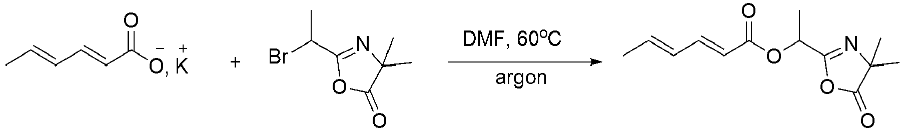

### 4.1. 2-(1-bromoéthyl)-4,4-diméthyloxazol-5(4H)-one

Elle est préparée selon la procédure décrite par K.M. Lewandowski *et al.* dans le brevet US 6 762 257 B1 (2004).

### 4.2. 1-(4,4-diméthyl-5-oxo-4,5-dihydrooxazol-2-yl)éthylhexa-2,4-dienoate

Dans un ballon muni d'un barreau aimanté sont introduits le sorbate de potassium (1,12 g, 7,5x10⁻³ mol), le 2-(1-bromoéthyl)-4,4-diméthyloxazol-5(4H)-one (1,68 g, 7,6x10⁻³ mol) et le DMF anhydre (20,0 mL). Ensuite, le mélange réactionnel est agité et chauffé à 60°C sous argon pendant 17 h. Le solvant (DMF) est éliminé sous pression réduite. Le résidu est repris par l'acétone puis filtré sur fritté. Le filtrat obtenu est concentré sous vide. Le produit final est obtenu sous forme d'une huile jaune foncée avec un rendement de 95% (1,90 g) qui cristallise au froid.
RMN ¹H (400 MHz, acétone D₆, δ ppm) : 1,40 ppm (6H, -C(CH₃)₂), 1,59 ppm (d, 3H, -COOCH(CH₃)), 1,90 ppm (d, 3H, H₃C-CH₂=CH₂-), 5,63 ppm (q, 1H, -CH(CH₃)OCO), 5,91 (d, 1H, CH₃-CH=CH-CH=CH-), 6,34 ppm (CH₃-CH=CH-CH=CH-), 7,34 ppm (1H, CH₃-CH=CH-CH=CH-).
RMN ¹³C (400 MHz, acétone D₆, δ ppm) : 17.01 ppm (-COOCH(CH₃)-), 18,51 ppm (H₃C-CH₂=CH₂-), 24,21 ppm et 24,31 ppm (C(CH₃)₂), 65,04 ppm (C(CH₃)₂), 65,85 ppm (-COO-CH(CH₃)-), 118,47 (CH₃-CH=CH-CH=CH-), 130,31 (CH₃-CH=CH-CH=CH-), 141,03 ppm (CH₃-CH=CH-CH=CH-), 146,88 ppm (CH₃-CH=CH-CH=CH-), 162,21 ppm (C=N), 165,92 ppm (-COO-CH(CH₃)-), 181,16 ppm (C=O)_{cycle}.
HR-MS : C₁₃H₁₇NO₄ m/z= [M+H]ₑₓₚ= 252,1239 ([M+H]_{cal}= 252,1236). FT-IR: v(CH) = 2985-2939, v(C=O)azlactone = 1826 cm⁻¹, v(C=O)ester = 1720 cm⁻¹, v(C=N) = 1683 cm⁻¹.

### Exemple 5 : Synthèse de la 2-(2-azidopropan-2-yl)-4,4-diméthyloxazol-5(4H)-one

La 2-(2-azidopropan-2-yl)-4,4-diméthyloxazol-5(4H)-one est synthétisée selon le schéma ci-dessous :

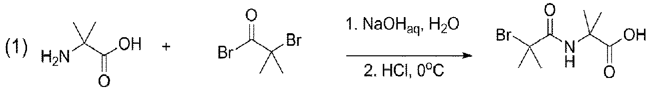

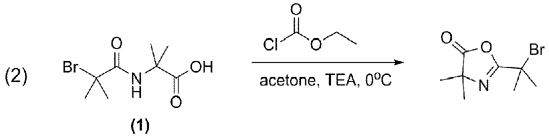

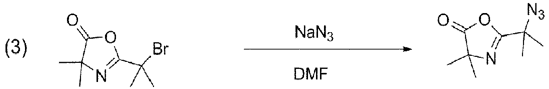

### 5.1. 2-(2-bromopropan-2-yl)-4,4-diméthyloxazol-5(4H)-one

Cet intermédiaire est préparé selon la procédure décrite par K.M. Lewandowski *et al.* dans le brevet US 6 894 133 (étapes 1 et 2)

### 5.2. 2-(2-azidopropan-2-yl)-4,4-diméthyloxazol-5(4H)-one

Elle est obtenue (étape 3) selon la procédure suivante :
Dans un ballon 25 mL muni d'un barreau aimanté, on prépare sous atmosphère d'argon une solution de 0,65 g (0,01 mol) d'azoture de sodium dans 5 mL de DMF anhydre que l'on refroidit à l'aide d'un bain de glace. On verse goutte-à-goutte sur ce mélange une solution comprenant 2,34 g, (0,01 mol) de 2-(2-bromopropan-2-yl)-4,4-diméthyloxazol-5(4H)-one dans 2 mL de DMF anhydre. Le mélange réactionnel est agité pendant 2 h à 0 °C puis à température ambiante pendant 16 h. Le solvant est évaporé sous pression réduite. Le résidu est repris par l'acétate d'éthyle puis la solution est filtrée pour éliminer le bromure de sodium. Le filtrat obtenu est concentré sous vide. L'huile jaune obtenue est purifiée par chromatographie sur colonne de silice avec successivement le *n*-hexane puis un mélange *n-*hexane : acétate d'éthyle 6:4 v/v pour fournir une huile incolore (0,84 g, 43 %).
RMN ¹H (200 MHz, CDCl₃, δ ppm) : 1,44 ppm (s, 6H, -C(CH₃)₂), 1,56 ppm (s, 6H, N₃C(CH₃)₂).
RMN ¹³C (400 MHz, CDCl₃, δ ppm) : 24,01 ppm (N₃C(CH₃)₂), 24,58 ppm (-C(CH₃)₂), 59,26 ppm (N₃C(CH₃)₂), 65,83 ppm (-C(CH₃)₂), 163,88 ppm (C=N), 180,41 ppm (C=O).

### Exemple 6 : Procédé de couplage du 1-(4,4-diméthyl-5-oxo-4,5-dihydrooxazol-2-yl)éthyl hex-5-ynoate avec le lysozyme

Dans un ballon équipé d'un barreau magnétique est préparée une solution de lysozyme (228,4 mg, 16,0x10⁻⁶ mol) dans le diméthylsulfoxide (DMSO, 20,00 mL) à laquelle on ajoute de la triéthylamine (TEA, 0,30 g, 3,0x10⁻³ mol). Après 30 min d'agitation à température ambiante, une solution de 1-(4,4-diméthyl-5-oxo-4,5-dihydrooxazol-2-yl)éthyl hex-5-ynoate (0,695 g, 3,0x10⁻³ mol) dans le DMSO (4,00 mL) est ajoutée et le mélange réactionnel est agité à température ambiante pendant 24 h. Après 24 h, un mélange d'HCl concentré (37%, 2 mL) et de 90 mL d'eau pure est ajouté, puis la solution est dialysée avec une membrane de dialyse (seuil de coupure = MWCO = 3500) contre une solution aqueuse de méthanol (eau : méthanol = 8 : 2 en volumes) pendant 24 h. Le produit est ensuite récupéré par la lyophilisation.

Le produit final est analysé par spectrométrie de masse à temps de vol (MALDI-TOF MS). Le résultat d'analyse de MALDI-TOF montre qu'après la réaction le pic du lysozyme de départ à m/z= 14357 a disparu au profit d'un nouveau signal centré à m/z= 17819.

## Revendications

1. Composé répondant à la formule générale (I) 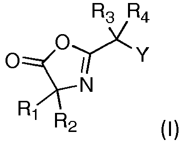 dans laquelle
- R₁ et R₂ représentent indépendamment l'un de l'autre un groupe (C₁-C₁₀)alkyle, un groupe (C₂-C₆)cycloalkyle, un groupe aryle, un groupe aryl(C₁-C₁₀)alkyle, ou un groupe hétérocyclique,
- R₃ et R₄ représentent indépendamment l'un de l'autre un atome d'hydrogène, un groupe (C₁-C₁₀)alkyle, un groupe aryle ou un groupe aryl(C₁-C₁₀)alkyle, et
- Y représente :
(i) un groupe N₃, ou
(ii) un groupe (C₁-C₁₀)alkyl-N₃, ou
(iii) un groupe aryl-N₃, ou
(iv) un groupe aryl(C₁-C₁₀)alkyl-N₃, ou
(v) un groupe (C₁-C₁₀)alkyl-C≡C-R₅, ou
(vi) un groupe aryl-C≡C-R₅, ou
(vii) un groupe aryl(C₁-C₁₀)alkyl- C≡C-R₅, ou
(viii) un groupe -O-C(O)-(CH₂)ₙ-C≡CR₅, avec n entier compris entre 1 et 10, notamment un groupe -O-C(O)-(CH₂)ₙ-C≡CH, avec n compris entre 1 et 5,
avec R₅ représentant
(a) soit un atome d'hydrogène,
(b) soit un groupe (C₁-C₁₀)alkyle,
(c) soit un groupe aryle,
(d) soit un groupe aryl(C₁-C₁₀)alkyle,
tous ces groupes pouvant renfermer deux doubles liaisons conjuguées, éventuellement dans un cycle,
(e) soit un groupe protecteur de la triple liaison.

2. Composé selon la revendication 1 dans lequel R₁ et R₂ représentent chacun un groupe (C₁-C₁₀)alkyle, R₃ représente un atome d'hydrogène, R₄ représente un groupe méthyle, et Y représente soit un groupe N₃, soit un groupe -O-C(O)-(CH₂)₃-C≡CH.

3. Composé selon la revendication 2 dans lequel R₁, R₂ et R₄ représentent chacun un groupe méthyle, R₃ un atome d'hydrogène et Y un groupe N₃.

4. Composé selon la revendication 2 dans lequel R₁, R₂ et R₄ désignent chacun un groupe méthyle, R₃ un atome d'hydrogène et Y un groupe -O-C(O)-(CH₂)₃-C≡CH.

5. Procédé de couplage entre une biomolécule et une molécule cible choisie dans le groupe comprenant une molécule d'intérêt biologique, un polymère naturel ou synthétique, et une surface réactive, **caractérisé en ce qu'**il met en oeuvre un composé de formule (I) 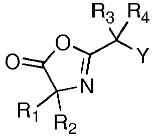 dans laquelle
- R₁ et R₂ représentent indépendamment l'un de l'autre un groupe (C₁-C₁₀)alkyle, un groupe (C₂-C₆)cycloalkyle, un groupe aryle, un groupe aryl(C₁-C₁₀)alkyle, ou un groupe hétérocyclique,
- R₃ et R₄ représentent indépendamment l'un de l'autre un atome d'hydrogène, un groupe (C₁-C₁₀)alkyle, un groupe aryle ou un groupe aryl(C₁-C₁₀)alkyle, et
- Y représente une fonction réactive activable par chimie click, choisie dans le groupe comprenant les azotures, les alcynes, les cycloalcynes et les diènes conjugués, en particulier un composé selon l'une quelconque des revendications 1 à 4 et **en ce qu'**il comprend les étapes suivantes :
- mise en contact dudit composé avec une biomolécule,
- mise en contact dudit composé lié avec la biomolécule avec la molécule cible et si nécessaire,
- isolement du produit de couplage.

6. Procédé selon la revendication 5 **caractérisé en ce que** la/les biomolécules mise(s) en jeu dans ledit procédé de couplage sont choisies dans le groupe comprenant des protéines, des peptides, de l'ADN, des marqueurs biologiques, des hormones, des vitamines, des anticorps, des polyamines, des monosaccharides, des oligosaccharides et des polysaccharides, et des molécules d'intérêt biologique du type médicament ou marqueur.

7. Réactif de diagnostic **caractérisé en ce qu'**il met en oeuvre au moins un composé selon l'une quelconque des revendications 1 à 4.

8. Utilisation d'un composé de formule (I) telle que représentée dans la revendication 1 dans laquelle
- R₁ et R₂ représentent indépendamment l'un de l'autre un groupe (C₁-C₁₀)alkyle, un groupe (C₂-C₆)cycloalkyle, un groupe aryle, un groupe aryl(C₁-C₁₀)alkyle, ou un groupe hétérocyclique,
- R₃ et R₄ représentent indépendamment l'un de l'autre un atome d'hydrogène, un groupe (C₁-C₁₀)alkyle, un groupe aryle ou un groupe aryl(C₁-C₁₀)alkyle, et
- Y représente une fonction réactive activable par chimie click, choisie dans le groupe comprenant les azotures, les alcynes, les cycloalcynes et les diènes conjugués,
comme réactif de diagnostic.

9. Kit pour la mise en oeuvre d'un procédé de couplage et de bioconjugaison selon l'une quelconque des revendications 5 et 6 **caractérisé en ce qu'**il comprend au moins un composé selon l'une quelconque des revendications 1 à 4.

10. Procédé de séparation, de détection et/ou de caractérisation d'au moins une molécule d'intérêt susceptible d'être présente dans un milieu, **caractérisé en ce qu'**il comprend au moins une étape de mise en oeuvre d'un kit selon la revendication 9 ou au moins une étape de mise en oeuvre d'un composé de formule (I) 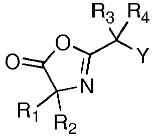 dans laquelle
- R₁ et R₂ représentent indépendamment l'un de l'autre un groupe (C₁-C₁₀)alkyle, un groupe (C₂-C₆)cycloalkyle, un groupe aryle, un groupe aryl(C₁-C₁₀)alkyle, ou un groupe hétérocyclique,
- R₃ et R₄ représentent indépendamment l'un de l'autre un atome d'hydrogène, un groupe (C₁-C₁₀)alkyle, un groupe aryle ou un groupe aryl(C₁-C₁₀)alkyle, et
- Y représente une fonction réactive activable par chimie click, choisie dans le groupe comprenant les azotures, les alcynes, les cycloalcynes et les diènes conjugués.

11. Composition comprenant un composé selon l'une quelconque des revendications 1 à 4 en association avec un milieu aqueux ou organique.

## Patentansprüche

1. Verbindung nach der allgemeinen Strukturformel (I) 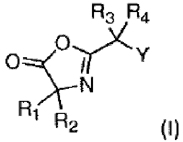 in welcher
- R₁ und R₂ unabhängig von einander eine (C₁-C₁₀)-Alkylgruppe, eine (C₂-C₆)-Cyclo-alkylgruppe, eine Arylgruppe, eine (C₁-C₁₀)-Alkylarylgruppe oder eine heterocyclische Gruppe darstellen,
- R₃ und R₄ unabhängig von einander ein Wasserstoffatom, eine (C₁-C₁₀)-Alkylgruppe, eine Arylgruppe oder eine (C₁-C₁₀)-Alkylarylgruppe darstellen, und
- Y für:
(i) eine N₃-Gruppe, oder
(ii) eine N₃-(C₁-C₁₀)-Alkylgruppe, oder
(iii) eine N₃-Arylgruppe, oder
(iv) eine N₃-(C₁-C₁₀)-Alkylarylgruppe, oder
(v) eine (C₁-C₁₀)Alkyl-C≡C-R₅-Gruppe, oder
(vi) eine Aryl-C≡C-R₅-Gruppe, oder
(vii) eine (C₁-C₁₀)Alkylaryl-C≡C-R₅-Gruppe, oder
(viii) eine -O-C(O)-(CH₂)ₙ-C≡CR₅-Gruppe
steht,
wobei n eine ganze Zahl zwischen 1 und 10 ist, insbesondere eine -O-C(O)-(CH₂)ₙ-C≡CH-Gruppe, wobei n zwischen 1 und 5 liegt,
wobei R₅ für
(a) entweder ein Wasserstoffatom,
(b) oder eine (C₁-C₁₀)Alkylgruppe,
(c) oder eine Arylgruppe,
(d) oder eine (C₁-C₁₀)Alkylarylgruppe,
wobei all diese Gruppen zwei konjugierte Doppelbindungen gegebenenfalls in einem Ring aufweisen können,
(e) oder für eine die Dreifachbindung schützende Gruppe
steht.

2. Verbindung nach Anspruch 1, wobei R₁ und R₂ jeweils eine (C₁-C₁₀)Alkylgruppe, R₃ ein Wasserstoffatom, R₄ eine Methylgruppe und Y entweder eine N₃-Gruppe oder eine -O-C(O)-(CH₂)₃-C≡CH-Gruppe darstellen.

3. Verbindung nach Anspruch 2, wobei R₁, R₂ und R₄ jeweils eine Methylgruppe, R₃ ein Wasserstoffatom und Y eine N₃-Gruppe darstellen.

4. Verbindung nach Anspruch 2, wobei R₁, R₂ und R₄ jeweils eine Methylgruppe, R₃ ein Wasserstoffatom und Y eine -O-C(O)-(CH₂)₃-C≡CH-Gruppe darstellen.

5. Verfahren zur Verknüpfung eines Biomoleküls mit einem Zielmolekül aus der Gruppe bestehend aus einem Molekül von biologischer Bedeutung, einem natürlichen oder synthetischen Polymer und einer reaktiven Oberfläche, **dadurch gekennzeichnet, dass** es eine Verbindung der Strukturformel (I) 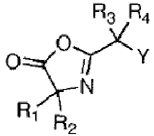 einsetzt, in welcher
- R₁ und R₂ unabhängig von einander eine (C₁-C₁₀)-Alkylgruppe, eine (C₂-C₆)-Cyclo-alkylgruppe, eine Arylgruppe, eine (C₁-C₁₀)-Alkylarylgruppe oder eine heterocyclische Gruppe darstellen,
- R₃ und R₄ unabhängig von einander ein Wasserstoffatom, eine (C₁-C₁₀)-Alkylgruppe, eine Arylgruppe oder eine (C₁-C₁₀)-Alkylarylgruppe darstellen, und
- Y für eine durch Click-Chemie bewirkbare Reagensfunktion steht, welche aus der Gruppe bestehend aus den Aziden, Alkinen, Cycloalkinen und konjugierten Dienen stammt, insbesondere aus einer Verbindung nach einem der Ansprüche 1-4, und dadurch, dass es folgende Schritte umfasst:
- Kontaktierung der Verbindung mit einem Biomolekül,
- Kontaktierung der mit dem Biomolekül gebundenen Verbindung mit dem Zielmolekül, und falls erforderlich
- Isolierung des Ergebnisses aus der Verknüpfung.

6. Verfahren nach Anspruch 5, **dadurch gekennzeichnet, dass** das/die im Verknüpfungs-verfahren eingesetzte(n) Biomolekül(e) aus der Gruppe bestehend aus Proteinen, Peptiden, DNA, Biomarkern, Hormonen, Vitaminen, Antikörpern, Polyaminen, Monosacchariden, Oligosacchariden und Polysacchariden, sowie Molekülen von biologischer Bedeutung vom Typ Medikament oder Marker ausgewählt wird/werden.

7. Diagnosereagens, **dadurch gekennzeichnet, dass** es mindestens eine Verbindung nach einem der Ansprüche 1-4 einsetzt.

8. Anwendung einer Verbindung der wie in Anspruch 1 dargestellten Strukturformel (I), in der
- R₁ und R₂ unabhängig von einander eine (C₁-C₁₀)-Alkylgruppe, eine (C₂-C₆)-Cyclo-alkylgruppe, eine Arylgruppe, eine (C₁-C₁₀)-Alkylarylgruppe oder eine heterocyclische Gruppe darstellen,
- R₃ und R₄ unabhängig von einander ein Wasserstoffatom, eine (C₁-C₁₀)-Alkylgruppe, eine Arylgruppe oder eine (C₁-C₁₀)-Alkylarylgruppe darstellen, und
- Y eine durch Click-Chemie bewirkbare Reagensfunktion darstellt, die aus der Gruppe bestehend aus den Aziden, Alkinen, Cycloalkinen und konjugierten Dienen stammt,
als Diagnosereagens.

9. Set für die Anwendung eines Verfahrens zur Verknüpfung und Biokonjugationen nach einem der Ansprüche 5-6, **dadurch gekennzeichnet, dass** es mindestens eine Verbindung nach einem der Ansprüche 1-4 umfasst.

10. Verfahren zur Trennung, Erkennung und/oder Charakterisierung mindestens eines bedeutenden, möglicherweise in einem Milieu vorkommenden Moleküls, **dadurch gekennzeichnet, dass** es mindestens einen Schritt zur Anwendung eines Sets nach Anspruch 9 oder zumindest einen Schritt zur Anwendung einer Verbindung mit der Strukturformel (I) 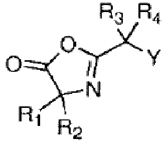 umfasst, in welcher
- R₁ und R₂ unabhängig von einander eine (C₁-C₁₀)-Alkylgruppe, eine (C₂-C₆)-Cycloalkylgruppe, eine Arylgruppe, eine (C₁-C₁₀)-Alkylarylgruppe oder eine heterocyclische Gruppe darstellen,
- R₃ und R₄ unabhängig von einander ein Wasserstoffatom, eine (C₁-C₁₀)-Alkylgruppe, eine Arylgruppe oder eine (C₁-C₁₀)-Alkylarylgruppe darstellen, und
- Y eine durch Click-Chemie bewirkbare Reagensfunktion darstellt, die aus der Gruppe bestehend aus den Aziden, Alkinen, Cycloalkinen und konjugierten Dienen stammt,

11. Zusammensetzung, umfassend eine Verbindung nach einem der Ansprüche 1-4 in Kombination mit einem wässrigen oder organischen Milieu.

## Claims

1. Compound corresponding to general formula (I) 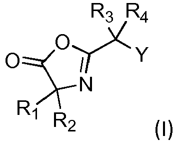 in which
- R₁ and R₂ represent independently of one another a (C₁-C₁₀)alkyl group, a (C₂-C₆)cycloalkyl group, an aryl group, an aryl(C₁-C₁₀)alkyl group, or a heterocyclic group,
- R₃ and R₄ represent independently of one another a hydrogen atom, a (C₁-C₁₀)alkyl group, an aryl group or an aryl(C₁-C₁₀)alkyl group, and
- Y represents:
(i) an N₃ group, or
(ii) a (C₁-C₁₀)alkyl-N₃ group, or
(iii) an aryl-N₃ group, or
(iv) an aryl(C₁-C₁₀)alkyl-N₃ group, or
(v) a (C₁-C₁₀)alkyl-C≡C-R₅ group, or
(vi) an aryl-C≡C-R₅ group, or
(vii) an aryl(C₁-C₁₀)alkyl-C≡C-R₅ group, or
(viii) an -O-C(O)-(CH₂)ₙ-C≡CR₅ group, with n an integer comprised between 1 and 10, in particular a -O-C(O)-(CH₂)ₙ-C≡CH group, with n comprised between 1 and 5,
with R₅ representing
(a) either a hydrogen atom,
(b) or a (C₁-C₁₀)alkyl group,
(c) or an aryl group,
(d) or an aryl(C₁-C₁₀)alkyl group,
all these groups being able to contain two conjugated double bonds, optionally in a ring,
(e) or a protective group of the triple bond

2. Compound according to claim 1 in which R₁ and R₂ each represent a (C₁-C₁₀)alkyl group, R₃ represents a hydrogen atom, R₄ represents a methyl group, and Y represents either an N₃ group, or an -O-C(O)-(CH₂)₃-C≡CH group.

3. Compound according to claim 2 in which R₁, R₂ and R₄ each represent a methyl group, R₃ a hydrogen atom and Y an N₃ group.

4. Compound according to claim 2 in which R₁, R₂ and R₄ each denote a methyl group, R₃ a hydrogen atom and Y an -O-C(O)-(CH₂)₃-C≡CH group.

5. Method for coupling a biomolecule and a target molecule selected from the group comprising a molecule of biological interest, a naturel or synthetic polymer, and a reactive surface, **characterized in that** it utilizes a compound of formula (I) 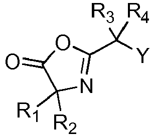 in which
- R₁ and R₂ represent independently of one another a (C₁-C₁₀)alkyl group, a (C₂-C₆)cycloalkyl group, an aryl group, an aryl(C₁-C₁₀)alkyl group, or a heterocyclic group,
- R₃ and R₄ represent independently of one another a hydrogen atom, a (C₁-C₁₀)alkyl group, an aryl group or an aryl(C₁-C₁₀)alkyl group, and
- Y represents a reactive function which can be activated by click chemistry, selected from the group comprising the azides, alkynes, cycloalkynes and conjugated dienes, in particular a compound according to any one of claims 1 to 4 and **in that** it comprises the following steps:
- bringing said compound into contact with a biomolecule,
- bringing said compound bound to the biomolecule into contact with the target molecule and if necessary,
- isolating the coupling product.

6. Method according to claim 5 **characterized in that** the biomolecule(s) involved in said coupling method are selected from the group comprising proteins, peptides, DNA, biological markers, hormones, vitamins, antibodies, polyamines, monosaccharides, oligosaccharides and polysaccharides, and molecules of biological interest of medicinal or label type.

7. Diagnostic reagent **characterized in that** it utilizes at least one compound according to any one of claims 1 to 4.

8. Use of a compound of formula (I) as described in claim 1, in which
- R₁ and R₂ represent independently of one another a (C₁-C₁₀)alkyl group, a (C₂-C₆)cycloalkyl group, an aryl group, an aryl(C₁-C₁₀)alkyl group, or a heterocyclic group,
- R₃ and R₄ represent independently of one another a hydrogen atom, a (C₁-C₁₀)alkyl group, an aryl group or an aryl(C₁-C₁₀)alkyl group, and
- Y represents a reactive function which can be activated by click chemistry, selected from the group comprising the azides, alkynes, cycloalkynes and conjugated dienes,
as a diagnostic reagent.

9. Kit for the implementation of a coupling and bioconjugation method according to any one of claims 5 and 6 **characterized in that** it comprises at least one compound according to any one of claims 1 to 5.

10. Method for the separation, detection and/or characterization of at least one molecule of interest potentially present in a medium, **characterized in that** it comprises at least one step of utilizing a kit according to claim 9 or at least one step of utilizing a compound of formula (I) 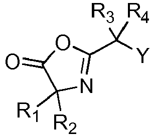 in which
- R₁ and R₂ represent independently of one another a (C₁-C₁₀)alkyl group, a (C₂-C₆)cycloalkyl group, an aryl group, an aryl(C₁-C₁₀)alkyl group, or a heterocyclic group,
- R₃ and R₄ represent independently of one another a hydrogen atom, a (C₁-C₁₀)alkyl group, an aryl group or an aryl(C₁-C₁₀)alkyl group, and
- Y represents a reactive function which can be activated by click chemistry, selected from the group comprising the azides, alkynes, cycloalkynes and conjugated dienes.

11. Composition comprising a compound according to any one of claims 1 to 5 in combination with an aqueous or organic medium.
